# EUROPEAN PATENT SPECIFICATION

(11) **EP 3 248 640 B1**
(45) Date of publication and mention of the grant of the patent: **11.03.2020**
(21) Application number: 16740152.0
(22) Date of filing: 19.01.2016
(51) Int. Cl.: A61J 1/20, A61M 5/34, A61M 39/10

(54) **MEDICAL LIQUID-COLLECTION INJECTOR**
MEDIZINISCHER FLÜSSIGKEITSSAMMELINJEKTOR
COLLECTEUR/INJECTEUR DE LIQUIDE À USAGE MÉDICAL

(30) Priority: 19.01.2015 JP 2015008090
(43) Date of publication of application: 29.11.2017
(73) Proprietor: JMS Co., Ltd., Hiroshima 730-8652 (JP)
(72) Inventor: YUKI, Takehiko, Hiroshima 730-8652 (JP); UEHARA, Yasumasa, Hiroshima 730-8652 (JP); KUNISHIGE, Takahiko, Hiroshima 730-8652 (JP); NAKANO, Kiyomi, Hiroshima 730-8652 (JP); UEHARA, Megumi, Hiroshima 730-8652 (JP); TAKIMOTO, Kazuhiko, Hiroshima 730-8652 (JP); ISHIDA, Miki, Hiroshima 730-8652 (JP)
(74) Representative: Dehns
(86) International application number: PCT/JP2016/051412
(87) International publication number: WO 2016/117545

(56) References cited:
- EP-A1- 1 872 824
- WO-A1-2004/096326
- JP-A- 2005 525 836
- JP-A- 2007 130 488
- JP-A- 2007 167 576
- JP-A- 2007 503 859
- JP-A- 2013 132 349

## Description

### Technical Field

The present invention relates to a medical liquid collection injector that can preferably be used when performing a simple suspension method.

### Background Art

Enteral nutrition therapy is known as a method for non-orally administering nutrition and medicine to a patient. In enteral nutrition therapy, a nasal catheter inserted into the stomach or duodenum through the nasal cavity of a patient, or a PEG (percutaneous endoscopic gastrostomy) catheter inserted into a gastric fistula formed in the stomach of a patient is used. A liquid such as nutrients, liquid food (commonly known as "enteral nutrients"), or medicine is administered to the patient via the nasal catheter or the PEG catheter (hereinafter collectively referred to as "catheter"). At the time of administering the liquid to the patient, a connector (hereinafter referred to as a "patient-side connector") provided on an upstream end of a catheter inserted into the patient, or provided on an upstream end of a flexible tube (commonly known as an "extension tube") that is connected to the catheter, and a connector (hereinafter referred to as a "container-side connector") connected to a container storing the liquid, or to a tube connected to the container, are connected. Conventionally, a female connector has been used as the patient-side connector and a male connector has been used as the container-side connector (e.g., see Patent Document 1).

In many cases, the patient into which the catheter is inserted cannot directly swallow medicine such as a tablet through the mouth. A "simple suspension method" is known as a method for administering medicine to the patient in such a case. The simple suspension method is performed using the following procedure. First, medicinal liquid obtained by disintegrating a tablet in lukewarm water or the like is formed in a container. Next, the medicinal liquid is suctioned into the injector (syringe). Next, the tube end of the injector is connected to the patient-side connector, and the medicinal liquid is administered to the patient via the catheter.

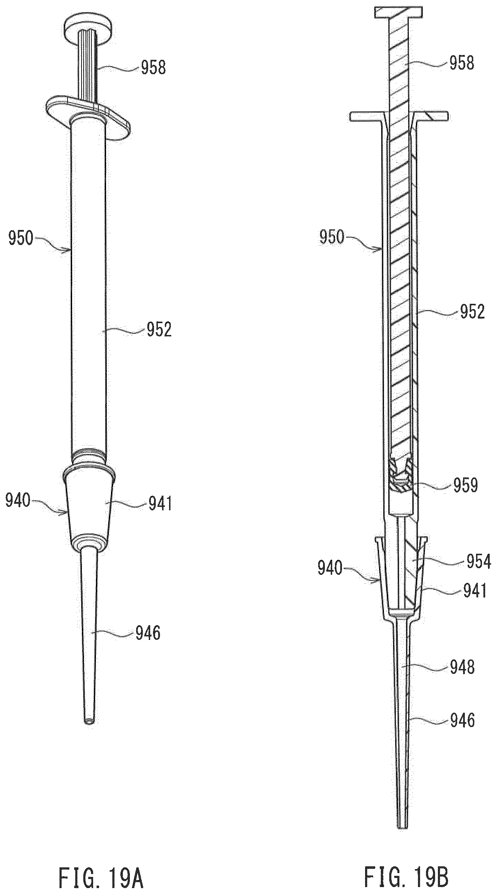

With the simple suspension method, it is necessary to connect the tube end of the injector to the patient-side connector. FIG. 19A is a perspective view of an injector 950 provided with a conventional liquid collection tip 940 used in the simple suspension method, and FIG. 19B is a cross-sectional view of the injector 950 provided with the liquid collection tip 940. Similarly to a general-purpose syringe, the injector 950 includes a barrel (outer tube) 952 and a plunger 958 that moves into and out of the barrel 952. The outer circumferential surface of the tube end (nozzle) 954 of the barrel 952 is a tapered surface (male tapered surface) with an outer diameter that becomes smaller as the leading end is approached. The liquid collection tip (hereinafter simply referred to as "tip") 940 is detachably mounted on the tube end 954. The tip 940 includes, at one end, a base end portion 941 that is to be fluid-tightly connected to the tube end 954, and includes a liquid collection needle 946 at the other end. A flow path 948 that penetrates through the tip 940 causes the base end portion 941 and the liquid collection needle 946 to be in communication. In the simple suspension method, in a state in which the tip 940 is mounted on the injector 950 as shown in FIGS. 19A and 19B, the medicinal liquid is suctioned into the barrel 952 while the leading end of the liquid collection needle 946 is immersed in the medicinal liquid. Thereafter, the tip 940 is removed from the injector 950, the tube end 954 is inserted into a patient-side connector (female connector), and the medicinal liquid in the barrel 952 is administered to the patient.

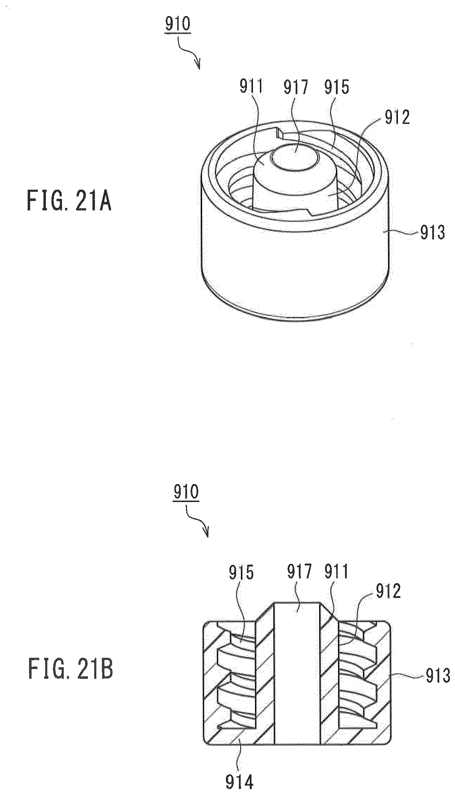

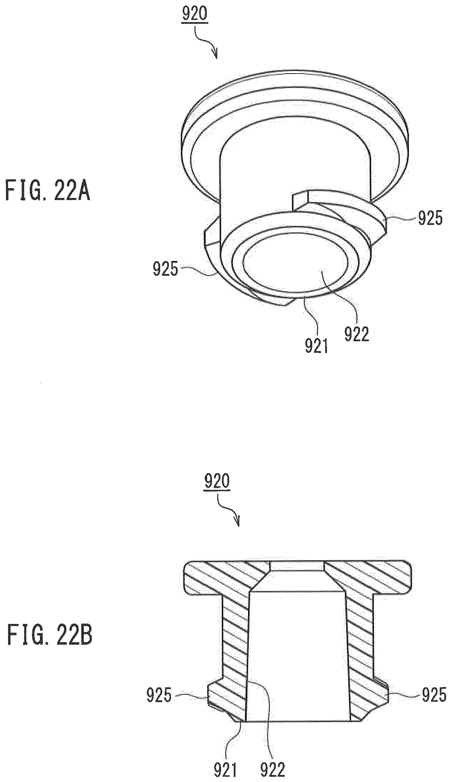

Incidentally, in recent years, consideration has been given to internationally standardizing, as International Standard ISO 80369-3 regarding nutritional medical devices, a male connector 910 shown in FIGS. 21A and 21B as a patient-side connector and a female connector 920 shown in FIGS. 22A and 22B as a container-side connector in order to prevent misconnection with a connector to be used in a field other than enteral nutrition.

The male connector (patient-side connector) 910 shown in FIGS. 21A and 21B includes a cylindrical male member 911 and an outer tube 913 that surrounds the male member 911. The male member 911 and the outer tube 913 are joined via a bottom plate 914 that protrudes in the form of a flange along the radial direction from the base end portion of the male member 911. The outer circumferential surface 912 of the male member 911 is a tapered surface (a so-called male tapered surface) with an outer diameter that becomes smaller as the leading end is approached. A flow path 917 that penetrates through the male member 911 is formed on the male member 911 along the lengthwise direction thereof. A female screw 915 is formed on the inner circumferential surface of the outer tube 913, which opposes the male member 911.

On the other hand, the female connector (container-side connector) 920 shown in FIGS. 22A and 22B includes a cylindrically-shaped tubular portion (female lure) 921 into which the male member 911 is inserted. The inner circumferential surface 922 of the tubular portion 921 is a tapered surface (so-called female tapered surface) with an inner diameter that becomes larger as the leading end is approached. A spiral projection (male screw) 925 is formed on the outer circumferential surface of the tubular portion 921.

The male connector 910 and the female connector 920 are connected due to the male member 911 being inserted into the tubular portion 921 and the female screw 915 and the spiral protrusion 925 being screwed together. The outer circumferential surface 912 of the male member 911 and the inner circumferential surface 922 of the tubular portion 921 are tapered surfaces with the same diameter and tapering angle, and therefore both come into fluid-tight surface contact with each other. The female screw 915 and the spiral projection 925 that are screwed together constitute a lock mechanism for locking the connected state of the male connector 910 and the female connector 920. The male connector 910 and the female connector 920 provide a connection with excellent fluid-tightness (a property of not allowing the liquid to leak from the portion at which the male connector and the female connector are connected, even if pressure is applied to the liquid) and connection strength (a property according to which the connected male connector and female connector do not separate even if a pulling force is applied thereto).

In the case where the patient-side connector is the male connector 910 shown in FIGS. 21A and 21B, the tube end 954 of the injector 950 shown in FIGS. 19A and 19B cannot be connected to the male connector 910. The tube end of the injector that is used in the simple suspension method needs to include the female connector 920 shown in FIGS. 22A and 22B in order to be able to connect to the male connector 910.

Patent Document 2 discloses a prefilled syringe with a spiral projection (male screw), and an adapter (needle) having a female screw that can be threadably engaged.

### Citation List

### Patent Documents

Patent Document 1: WO 2008/152871
Patent Document 2: JP 2007/167576 A

### Disclosure of Invention

### Problem to be Solved by the Invention

When considering an injector having the female connector 920 at its tube end, it is desirable to give consideration to the following two points.

Firstly, it is desirable that the medicinal liquid does not attach to the inner circumferential surface (in particular, the female screw 915) of the outer tube 913 of the male connector (patient-side connector) 910 via the injector.

As shown in FIGS. 21A and 21B, the female screw 915 is formed on the inner circumferential surface of the outer tube 913 of the male connector 910. When the medicinal liquid attaches to the troughs of the female screw 915, it is difficult to remove the medicinal liquid by wiping. If the male connector 910 is provided on the upstream-side end of the catheter inserted in the patient, the male connector 910 continues to be left in the patient along with the catheter for a long time in some cases. For example, a PEG catheter is commonly replaced every 1 to 3 months. If the medicinal liquid thus continues to be attached to the male connector 910 for a long time, the male connector 910 can reach an unhygienic state. Then, there is a possibility that bacteria will eventually reproduce in the male connector 910 and the bacteria will enter the body of the patient and cause serious complications.

If the tube end of the injector includes the female connector 920, the medicinal liquid attaches to the inner circumferential surface of the outer tube 913 of the male connector 910 when the female connector 920 is connected to the male connector 910 in the state in which the medicinal liquid is attached to the outer circumferential surface (in particular, the spiral projection 925) of the tubular portion 921. In order to prevent the male connector 910 from reaching an unhygienic state such as that described above, it is necessary to prevent the medicinal liquid from attaching to the inner circumferential surface (in particular, the female screw 915) of the outer tube 913 of the male connector 910 via the injector.

Secondly, it is desired that the medicinal liquid amount administered to the patient is accurately managed. The injector 950 provided with the conventional tip 940 shown in FIGS. 19A and 19B is problematic in that there are cases where the medicinal liquid amount cannot be accurately managed, depending on the operation method of the worker.

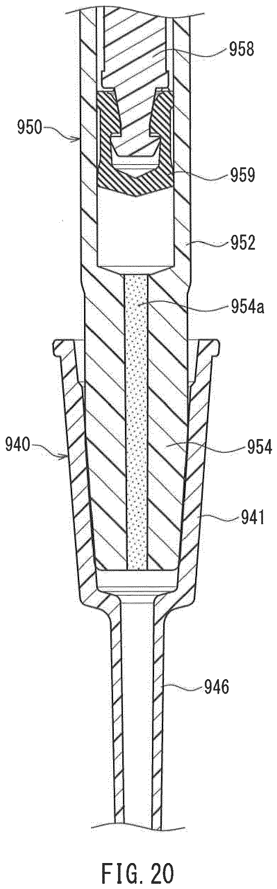

That is, in the case of performing the simple suspension method using the injector 950 provided with the conventional tip 940, as described with reference to FIGS. 19A and 19B, the medicinal liquid is suctioned into the barrel 952 in a state in which the tip 940 is attached to the injector 950. The suction amount of the medicinal liquid is measured using notches (not shown) on the outer circumferential surface of the barrel 952. FIG. 20 is an enlarged cross-sectional view showing the tube end 954 of the injector 950 on which the tip 940 is mounted, and the periphery thereof. In general, the volume indicated by the notches on the barrel 952 does not include the volume of the space 954a (the portion denoted by many dots in FIG. 20) in the tube end 954. This is because the gasket 959 provided on the leading end of the plunger 958 cannot advance into the space 954a. Accordingly, the simple suspension method needs to be ended in a state in which the plunger 958 is pressed into the barrel 952 until the gasket 959 hits the deepest portion of the barrel 952, and the medicinal liquid remains in the space 954a. If the medicinal liquid that is to remain in the space 954a in the tube end 954 is administered to the patient, the medicinal liquid will be administered in excess of the measured amount accordingly. For example, after the injector 950, which has suctioned the correct amount of medicinal liquid, is connected to the patient-side connector and the medicinal liquid in the barrel 952 has been administered to the patient, lukewarm water or the like is suctioned into the injector 950, and the medicinal liquid remaining in the space 954a is administered to the patient along with the lukewarm water, whereupon an amount of medicinal liquid that is greater than the measured amount is administered to the patient. In general, the person performing the simple suspension method is not limited to being a medical professional, and it is often the case that it is performed by a caregiver such as a family member of the patient. In such a case, there is a possibility that unsuitable medicinal liquid amount management such as that described above will be performed.

A first object of the present invention is to prevent a medicinal liquid from attaching to a female screw that surrounds a male member of a male connector when an injector is connected to the male connector in a simple suspension method. A second object of the present invention is to reduce the likelihood that an unsuitable amount of medicinal liquid will be administered to a patient in the simple suspension method.

### Means for Solving the Problem

A medical liquid collection injector of the present invention includes: a tube-shaped barrel that includes an opening at one end and includes a tube end at another end; a plunger that is inserted into the opening of the barrel; and a tube-shaped liquid collection needle. The medical liquid collection injector is characterised in that the tube end includes a cylindrically-shaped tubular portion and a spiral projection that is formed on an outer circumferential surface of the tubular portion. An inner circumferential surface of the tubular portion is a female tapered surface with an inner diameter that becomes larger as a leading end is approached. The barrel includes a small-diameter portion with an inner diameter that is relatively small between a liquid storing portion in which the plunger is inserted and the tubular portion. A base end of the liquid collection needle is fluid-tightly connected to the small-diameter portion, and a leading end of the liquid collection needle protrudes from the tube end.

### Effects of the Invention

With the medical liquid collection injector of the present invention, the base end of the liquid collection needle and the small-diameter portion of the barrel are fluid-tightly connected. Accordingly, in the case of performing a simple suspension method, the medicinal liquid does not attach to the outer circumferential surface of the tube end if the medicinal liquid is suctioned into the injector via the liquid collection needle. Thereafter, the liquid collection needle is removed from the tube end, and the tube end is connected to the male connector (patient-side connector). Accordingly, it is possible to prevent the medicinal liquid from attaching to the female screw surrounding the male member of the male connector.

Also, the medicinal liquid in the liquid storing portion does not pass between the base end of the liquid collection needle and the small-diameter portion of the barrel to leak out to the tube end side. Accordingly, it is possible to always administer a correct amount of the medicinal liquid to the patient.

### Brief Description of Drawings

[FIG. 1] FIG. 1A is a perspective view of a medical liquid collection injector according to Embodiment 1 of the present invention. FIG. 1B is a cross-sectional view of the medical liquid collection injector according to Embodiment 1 of the present invention.
[FIG. 2] FIG. 2 is an exploded perspective view of the medical liquid collection injector according to Embodiment 1 of the present invention.
[FIG. 3] FIG. 3A is a perspective view of a liquid collection needle included in the medical liquid collection injector according to Embodiment 1 of the present invention. FIG. 3B is a cross-sectional view of the liquid collection needle.
[FIG. 4] FIG. 4 is an enlarged cross-sectional view showing a portion at which the liquid collection needle and the tube end are connected in the medical liquid collection injector according to Embodiment 1 of the present invention.
[FIG. 5] FIG. 5A is a perspective view of a medical liquid collection injector according to Embodiment 2 of the present invention. FIG. 5B is a cross-sectional view of the medical liquid collection injector according to Embodiment 2 of the present invention.
[FIG. 6] FIG. 6 is an enlarged cross-sectional view showing a portion at which the liquid collection needle and the small-diameter portion are connected in the medical liquid collection injector according to Embodiment 2 of the present invention.
[FIG. 7] FIG. 7A is a perspective view of the medical liquid collection injector according to Embodiment 2 of the present invention, from which the liquid collection needle has been removed. FIG. 7B is a cross-sectional view of the medical liquid collection injector according to Embodiment 2 of the present invention, from which the liquid collection needle has been removed.
[FIG. 8] FIG. 8A is a perspective view of a medical liquid collection injector according to Embodiment 3 of the present invention. FIG. 8B is a cross-sectional view of the medical liquid collection injector according to Embodiment 3 of the present invention.
[FIG. 9] FIG. 9 is an exploded perspective view of the medical liquid collection injector according to Embodiment 3 of the present invention.
[FIG. 10] FIG. 10A is a perspective view from above of a liquid collection needle included in the medical liquid collection injector according to Embodiment 3 of the present invention. FIG. 10B is a perspective view from below of the liquid collection needle.
[FIG. 11] FIG. 11 is a cross-sectional perspective view of the liquid collection needle according to Embodiment 3 of the present invention.
[FIG. 12] FIG. 12 is a plan view of the liquid collection needle according to Embodiment 3 of the present invention.
[FIG. 13] FIG. 13A is a perspective view from above of a nozzle tip included in the medical liquid collection injector according to Embodiment 3 of the present invention. FIG. 13B is a perspective view from below of the nozzle tip.
[FIG. 14] FIG. 14 is a cross-sectional perspective view of the nozzle tip according to Embodiment 3 of the present invention.
[FIG. 15] FIG. 15A is a perspective view of a liquid collection nozzle in which the nozzle tip is mounted on the liquid collection needle in Embodiment 3 of the present invention. FIG. 15B is a cross-sectional view of the liquid collection nozzle.
[FIG. 16] FIG. 16A is a perspective view showing a state in which the liquid collection needle is mounted on an injector main body in Embodiment 3 of the present invention. FIG. 16B is a cross-sectional view of FIG. 16A.
[FIG. 17] FIG. 17 is a perspective view showing a state in which the medical liquid collection injector according to Embodiment 3 of the present invention has pierced an opening of a container.
[FIG. 18] FIG. 18 is a cross-sectional view showing another liquid collection nozzle in Embodiment 3 of the present invention.
[FIG. 19] FIG. 19A is a perspective view of an injector of an injector provided with a conventional liquid collection tip that is used in a simple suspension method. FIG. 19B is a cross-sectional view of the injector provided with the liquid collection tip.
[FIG. 20] FIG. 20 is an enlarged cross-sectional view showing a tube end of an injector and its periphery, on which a conventional tip is mounted.
[FIG. 21] FIG. 21A is a perspective view of a male connector (patient-side connector) that is being considered as ISO 80369-3. FIG. 21B is a cross-sectional view of the male connector taken along a plane including the central axis of the male connector.
[FIG. 22] FIG. 22A is a perspective view of a female connector (container-side connector) that is being considered as ISO 80369-3. FIG. 22B is a cross-sectional view of the female connector. taken along a plane including the central axis of the female connector.

### Description of the Invention

The barrel and the liquid collection needle may be separate parts in the medical liquid collection injector according to the above-described present invention. In this case, the liquid collection needle is preferably detachably mounted on the tube end. According to this preferable configuration, if the medicinal liquid amount in the container is large, the same injector can be used to administer the medicinal liquid to the patient by repeatedly mounting and removing the liquid collection needle on/from the tube end multiple times.

In the description above, it is preferable that a fitting shape that fits in the inner circumferential surface of the tubular portion is formed on the liquid collection needle. According to this preferable configuration, the liquid collection needle can be firmly held on the tube end.

The barrel and the liquid collection needle may constitute a part that is formed integrally. In this case, it is preferable that the liquid collection needle and the barrel can be irreversibly separated at a boundary between the base end and the small-diameter portion. According to this preferable configuration, the liquid collection needle can be removed from the barrel. Accordingly, thereafter, the tube end can be connected to the patient-side connector. Since the injector from which the liquid collection needle has been removed cannot be re-used, the likelihood that the patient will be infected due to re-use of the injector is low. Also, it is possible to reduce the number of parts that constitute the injector, which makes it easier to manufacture the injector.

The medical liquid collection injector of the above-described invention may further include a nozzle tip that is detachably mounted on the liquid collection needle, so as to cover at least the leading end of the liquid collection needle. In this case, it is preferable that the nozzle tip includes an opening that is provided so as to be in communication with a flow path of the liquid collection needle when the nozzle tip is mounted on the liquid collection needle. According to this preferred embodiment, an injector that can be used in the simple suspension method can be used to extract breast milk if the nozzle tip is mounted on the liquid collection needle.

It is preferable that an air-tight and fluid-tight seal is formed between the liquid collection needle and the nozzle tip when the nozzle tip is mounted on the liquid collection needle. According to this preferable configuration, breast milk can be extracted efficiently.

A lock mechanism for maintaining a state in which the nozzle tip is mounted on the liquid collection needle may be provided. According to this preferable configuration, it is possible to reduce the likelihood that the nozzle tip will unintentionally fall off of the liquid collection needle.

A male tapered surface with an outer diameter that becomes smaller as the leading end is approached may be provided on the outer circumferential surface of the nozzle tip. According to this preferred configuration, the injector can pierce the container in a state in which the male tapered surface has been fit into the edge of the opening of the container. This is advantageous for preventing contamination of the leading end of the nozzle tip and preventing contamination and evaporation of the liquid in the container.

It is preferable that the tubular portion, the spiral projection, and the female tapered surface conform to ISO 80369-3. According to this preferable configuration, the tube end of the injector can be connected to a male connector (patient-side connector) conforming to ISO 80369-3 with an air-tightness and connection strength that conform to ISO 80369-3.

Hereinafter, the present invention will be described in detail by means of preferred embodiments. However, it goes without saying that the present invention is not limited to the following embodiments. In the drawings referenced in the following description, only the relevant members needed in order to describe the present invention among the members constituting the embodiment of the present invention are shown in a simplified manner for the sake of convenience in the description. Accordingly, the present invention can include any member that is not shown in the following drawings. Also, in the following drawings, the actual dimensions of members and dimensional proportions of members and the like are not necessarily rendered faithfully. In the drawings shown below, identical members are denoted by identical reference signs, and redundant description thereof is not included.

### Embodiment 1

### Configuration

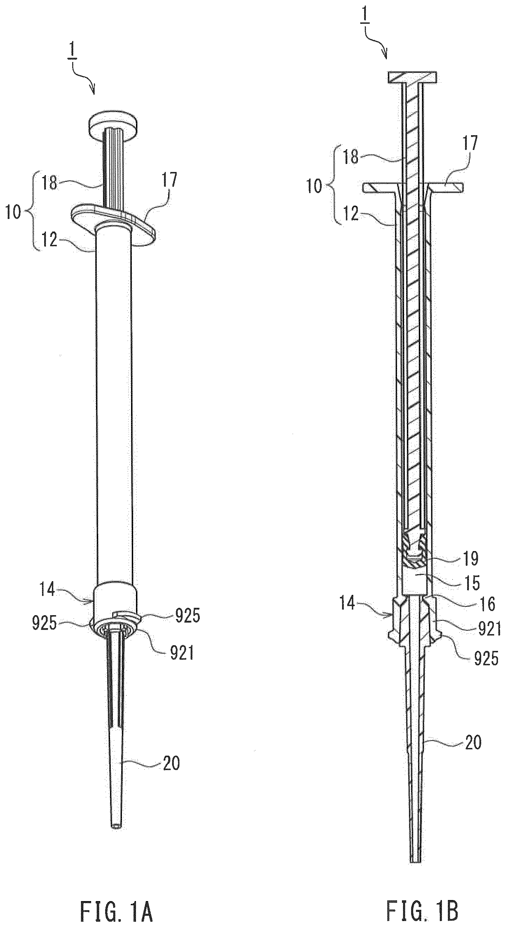

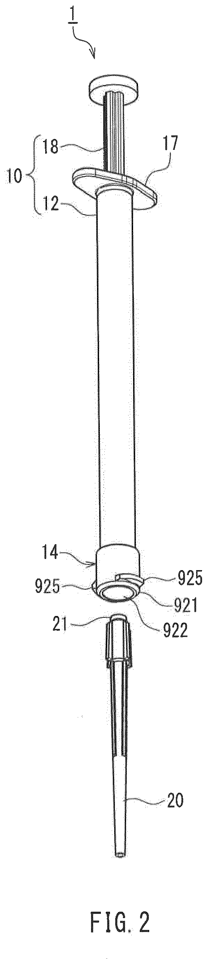

FIG. 1A is a perspective view of a medical liquid collection injector (hereinafter referred to as simply "injector") 1 according to Embodiment 1 of the present invention. FIG. 1B is a cross-sectional view of the injector 1. FIG. 2 is an exploded perspective view of the injector 1. The injector 1 includes an injector main body 10 having a barrel (outer tube) 12 and a plunger 18, and a liquid collection needle 20. For the sake of convenience in the following description, the plunger 18 side is referred to as the "upper" side of the injector 1 and the liquid collection needle 20 side is referred to as the "lower" side of the injector 1. Also, the direction in which the plunger 18 and the liquid collection needle 20 are connected is referred to as the "lengthwise direction" of the injector 1.

The barrel 12 has a hollow cylindrical shape, one end thereof (upper end) is open, and a tube end (nozzle) 14 is included at the other end (lower end). The plunger 18 is inserted into the opening at the upper end of the barrel 12 so as to be able to move in and out. A gasket 19 is attached to the leading end of the plunger 18. The gasket 19 slides in the lengthwise direction on the inner circumferential surface of the barrel 12 while forming a fluid-tight seal with the inner circumferential surface of the barrel 12. A pair of finger-hooking flanges 17 protrude outward from the upper end of the barrel 12. Notches (not shown) indicating a liquid amount in the barrel 12 are provided on the outer circumferential surface of the barrel 12.

A female connector that conforms to ISO 80369-3 and is the same as the female connector (container-side connector) shown in FIGS. 22A and 22B is formed on the tube end 14 of the barrel 12 so as to be able to connect to the male connector (patient-side connector) 910 (see FIGS. 21A and 21B). In FIGS. 1A, 1B, and 2, members that are the same as the members shown in FIGS. 22A and 22B are denoted by the same reference signs thereas. As shown in FIG. 1B, a small-diameter portion 16 having a smaller inner diameter than a liquid storing portion 15 and a tubular portion 921 is formed between the liquid storing portion 15 and the tubular portion 921. Here, the liquid storing portion 15 is a portion of the barrel 12 that the plunger 18 moves into and out of and is a portion that can store the medicinal liquid. The small-diameter portion 16 prevents the plunger 18 inserted into the liquid storing portion 15 from advancing into the tubular portion 921 of the tube end 14.

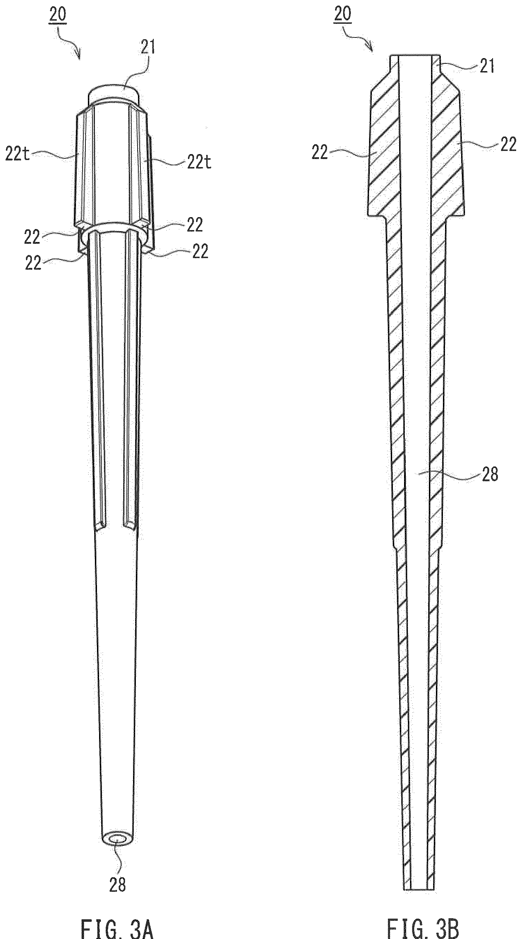

FIG. 3A is a perspective view of the liquid collection needle 20 and FIG. 3B is a cross-sectional view of the liquid collection needle 20. The liquid collection needle 20 has an elongated rod shape overall. A flow path 28 penetrates through the liquid collection needle 20 in the lengthwise direction thereof, and opens at the base end (end on the side that is to be inserted into the tube end 14 of the barrel 12) and the leading end of the liquid collection needle 20.

A connection tube 21 is formed at the base end of the liquid collection needle 20. The outer circumferential surface of the connection tube 21 is a cylindrical surface with a constant outer diameter in the lengthwise direction. Multiple (in this example, four) ribs 22 protrude outward in the radial direction from the outer circumferential surface of the liquid collection needle 20, adjacent to the connection tube 21. The ribs 22 extend along the lengthwise direction of the liquid collection needle 20. The outer diameter of the liquid collection needle 20 at the ribs 22 becomes smaller as the connection tube 21 is approached. More specifically, top surfaces (the surfaces facing outward in the radial direction of the ribs 22) 22t of the ribs 22 conform to the male tapered surface conforming to ISO 80369-3, which is formed on the outer circumferential surface 912 (see FIGS. 21A and 21B) of the male member 911 of the above-described male connector 910. In other words, the top surfaces 22t of the ribs 22 conform to a tapered surface (conical surface) having the same tapering angle and diameter as the female tapered surface formed on the inner circumferential surface 922 of the tubular portion 921 of the tube end 14.

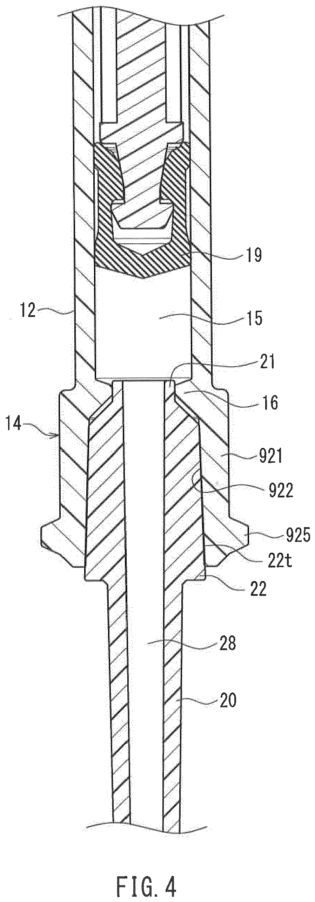

The connection tube 21 of the liquid collection needle 20 is inserted into the tube end 14 of the barrel 12. As shown in FIG. 4, the connection tube 21 of the liquid collection needle 20 fits in the small-diameter portion 16 of the barrel 12. The outer diameter of the connection tube 21 approximately matches the inner diameter of the small-diameter portion 16. Accordingly, a fluid-tight seal is formed between the outer circumferential surface of the connection tube 21 and the inner circumferential end of the small-diameter portion 16. The flow path 28 of the liquid collection needle 20 is in communication with the liquid storing portion 15.

The top surfaces 22t of the ribs 22 of the connection tube 21 come into contact with the inner circumferential surface 922 of the tubular portion 921 of the barrel 12. As described above, the top surfaces 22t conform to the male tapered surface, which is the same as the outer circumferential surface 912 (see FIGS. 21A and 21B) of the male member 911 of the male connector 910. Accordingly, the top surfaces 22t come into surface contact with the inner circumferential surface 922 of the tubular portion 921. For this reason, the rib 22 and the tubular portion 921 fit together, and the liquid collection needle 20 is firmly connected to the tube end 14. The leading end of the liquid collection needle 20 significantly protrudes from the tube end 14 (see FIG. 1A).

The liquid collection needle 20 merely fits in the tube end 14, and therefore it is possible to repeatedly attach and detach the liquid collection needle 20 to and from the tube end 14.

The material of the barrel 12, the plunger 18 (except for the gasket 19), and the liquid collection needle 20 is not limited but is preferably a material with a shape-holding property, and furthermore, is preferably a hard material (solid material) that has a mechanical strength (rigidity) according to which deformation substantially does not occur due to an external force. For example, it is possible to use a resin material such as polypropylene (PP), polycarbonate (PC), polyacetal (POM), polystyrene, polyamide, polyethylene, rigid polyvinyl chloride, or acrylonitrile-butadiene-styrene copolymer (ABS), and among these, polypropylene (PP), polyethylene, polycarbonate (PC), and acrylonitrile-butadiene-styrene copolymer (ABS) are preferable. The barrel 12, the plunger 18, and the liquid collection needle 20 can be formed integrally as one part overall through an extrusion molding method or the like, using the above-described resin material.

The material of the gasket 19 is not limited and for example, it is possible to use butyl rubber, isoprene rubber, styrene-based thermoplastic elastomer, or the like thereas.

### Method of Use (said method is not forming part of the invention)

The injector 1 can be used in the case of performing the above-described simple suspension method. A simple suspension method using the injector 1 is performed as follows.

Firstly, the injector 1 obtained by mounting the liquid collection needle 20 on the tube end 14 of the injector main body 10 is prepared as shown in FIGS. 1A and 1B.

Next, the leading end of the liquid collection needle 20 is immersed in the medicinal liquid in which a tablet has been disintegrated, the plunger 18 is operated, and the medicinal liquid is suctioned into the barrel 12. The suction amount of the medicinal liquid is measured using the position of the gasket 19, which can be seen through the barrel 12, and the notches (not shown) on the barrel 12.

Next, the liquid collection needle 20 is taken off of the tube end 14 (see FIG. 2).

Next, the tube end 14 is connected to the male connector 910 (see FIGS. 21A and 21B). The male connector 910 is a patient-side connector provided on the upstream-side end of a catheter inserted into the body of the patient, or provided on the upstream-side end of an elongated tube connected to the catheter. Since the tube end 14 is the female connector 920 conforming to ISO 80369-3, the tube end 14 and the male connector 910 are connected with a fluid-tightness and a connection strength conforming to ISO 80369-3. In this state, the plunger 18 is pressed into the barrel 12 until the gasket 19 hits the small-diameter portion 16, whereby the medicinal liquid in the liquid storing portion 15 is administered to the patient. Thereafter, the tube end 14 is separated from the male connector 910.

### Effect

According to the present Embodiment 1, the task (suctioning task) of suctioning the medicinal liquid in the container into the injector main body 10 is performed with the liquid collection needle 20 mounted on the tube end 14, and on the other hand, the task (administration task) of administering the medicinal liquid in the injector main body 10 to the patient is performed with the tube end 14 connected to the male connector 910 without using the liquid collection needle 20. In the suctioning task, the leading end of the liquid collection needle 20 is immersed in the medicinal liquid. Also, the connection tube 21 of the liquid collection needle 20 is fluid-tightly connected to the small-diameter portion 16 of the barrel 12. Accordingly, the medicinal liquid does not attach to the outer circumferential surface of the tubular portion 921, which includes the spiral projection 925. For this reason, when the tube end 14 is connected to the male connector 910 thereafter, the medicinal liquid does not attach to the inner circumferential surface (in particular, the female screw 915) of the outer tube 913 of the male connector 910. Accordingly, it is possible to prevent the male connector 910 from reaching an unhygienic state, even if the male connector 910 is left in the patient for a long time.

When the suctioning task of suctioning the medicinal liquid into the injector main body 10 is performed with the tube end 14 directly immersed in the medicinal liquid without mounting the liquid collection needle 20 on the tube end 14, the medicinal liquid will attach not only to the inner circumferential surface of the tubular portion 921 but also to the outer circumferential surface of the tubular portion 921 including the spiral projection 925. Thereafter, when the tube end 14 is connected to the male connector 910 (see FIGS. 21A and 21B), the medicinal liquid attached to the outer circumferential surface of the tubular portion 921 attaches to the female screw 915 formed on the inner circumferential surface of the outer tube 913 of the male connector 910. As described above, if the suctioning task is performed with the liquid collection needle 20 mounted on the tube end 14, it is possible to reliably prevent the medicinal liquid from attaching to the outer circumferential surface of the tubular portion 921 including the spiral projection 925.

As shown in FIG. 4, when the liquid collection needle 20 is mounted on the tube end 14, the connection tube 21 provided on the base end of the liquid collection needle 20 fits into the small-diameter portion 16 of the barrel 12. Since the connection tube 21 and the small-diameter portion 16 are fluid-tightly connected, the medicinal liquid in the liquid storing portion 15 does not pass between the connection tube 21 and the small-diameter portion 16 to leak to the tube end 14 side. Accordingly, unlike the case of using the conventional tip 940, in the case of performing the simple suspension method using the injector 1, the medicinal liquid does not remain in the tube end 14. For this reason, in the present Embodiment 1, the likelihood that an amount of the medicinal liquid that is greater than the measured amount will be administered to the patient is reduced, regardless of the worker. Accordingly, the injector 1 of the present Embodiment 1 is advantageous for accurately managing a medicinal liquid amount to be administered to the patient.

The liquid collection needle 20 can be repeatedly attached to and detached from the tube end 14. Accordingly, if the medicinal liquid amount to be administered to the patient is greater in comparison to the capacity of the injector main body 10, it is possible to administer the medicinal liquid to the patient using the same injector 1 by repeatedly mounting and removing the liquid collection needle 20 to and from the tube end 14 multiple times.

In the present Embodiment 1, the number of ribs 22 formed near the connection tube 21 of the liquid collection needle 20 is arbitrary. Any fitting shape other than that of the rib 22, which fits together with the inner circumferential surface (female tapered surface) 922 of the tubular portion 921, may be formed on the liquid collection needle 20. For example, the male tapered surface conforming to ISO 80369-3, which is formed on the outer circumferential surface 912 (see FIGS. 21A and 21B) of the male member 911 of the male connector 910, may be formed on the liquid collection needle 20. Alternatively, any projection that comes into contact with the inner circumferential surface 922 of the tubular portion 921 (e.g., a ring-shaped projection that is continuous in the circumferential direction) may be formed on the liquid collection needle 20.

Alternatively, the ribs 22 that fit in the inner circumferential surface 922 of the tubular portion 921 or a fitting shape that resembles it does not need to be formed on the liquid collection needle 20. In this case, the liquid collection needle 20 is held to the barrel 12 at the connection tube 21.

The outer tube and the female screw conforming to ISO 80369-3, which are the same as the outer tube 913 and the female screw 915 provided on the male connector 910 (see FIGS. 21A and 21B), may be provided on the liquid collection needle 20. In this case, similarly to a liquid collection needle 320 according to later-described Embodiment 3, it is possible to screw the female screw onto the spiral projection 925 of the tubular portion 921.

### Embodiment 2

### Configuration

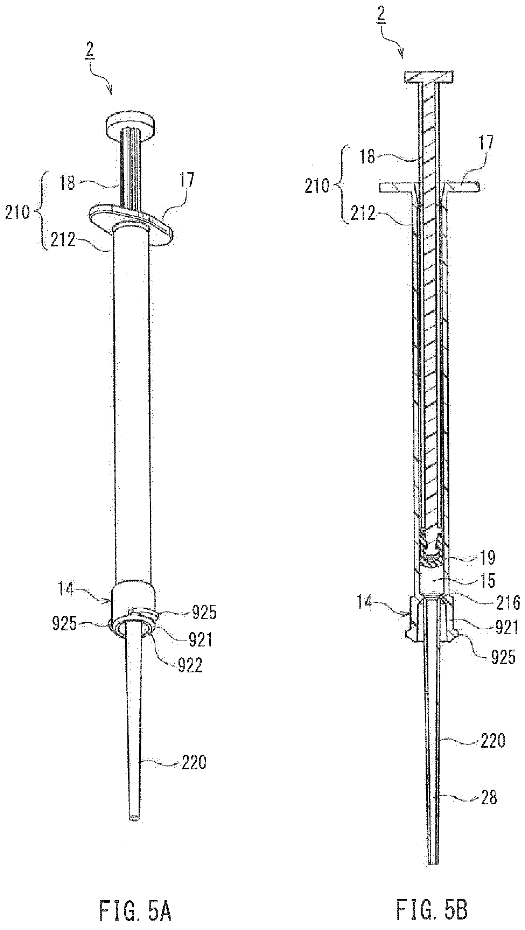

FIG. 5A is a perspective view of an injector 2 according to Embodiment 2 of the present invention. FIG. 5B is a cross-sectional view of the injector 2. The injector 2 of the present Embodiment 2 differs from the injector 1 of Embodiment 1 in which the liquid collection needle 20 and the barrel 12 were separate parts, in that it includes one part in which a liquid collection needle 220 is formed integrally with a barrel 212. Among the members constituting the injector 2 of Embodiment 2, members that are the same as the members constituting the injector 1 of Embodiment 1 are denoted by the same reference numerals as in Embodiment 1, and detailed description thereof is omitted.

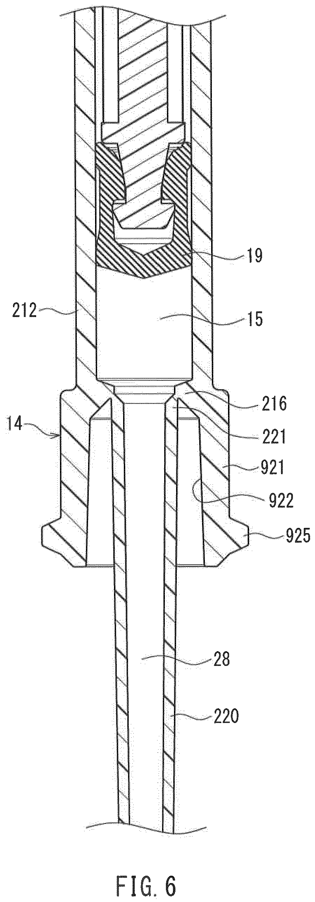

The liquid collection needle 220 of the present Embodiment 2 has an elongated rod shape overall, similarly to the liquid collection needle 20 of Embodiment 1. As shown in FIG. 5B, a flow path 28 penetrates through the liquid collection needle 220 in the lengthwise direction thereof and an opening is formed on the leading end of the liquid collection needle 220. As shown in FIG. 6, a base end 221 of the liquid collection needle 220 is connected to the small-diameter portion 216 of the barrel 212. The flow path 28 of the liquid collection needle 220 is in communication with the liquid storing portion 15.

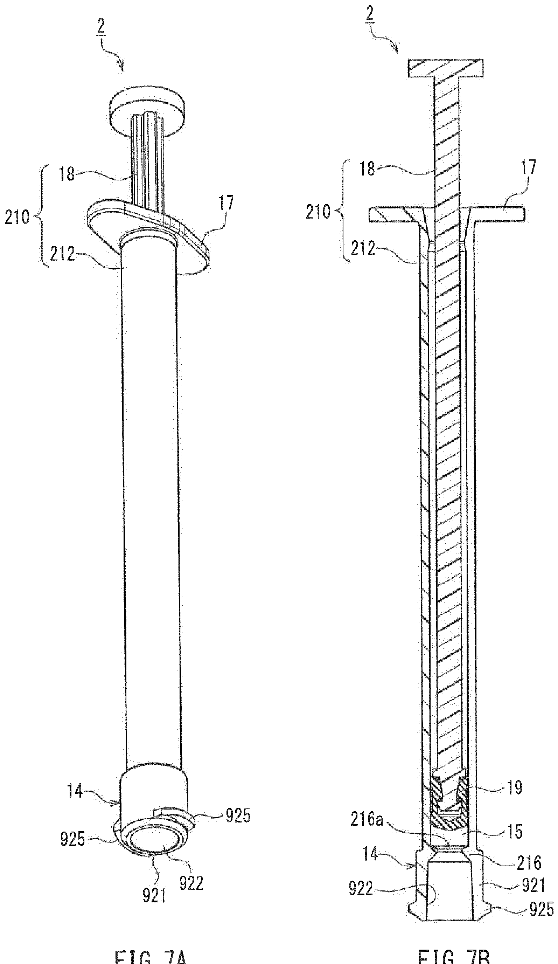

With the injector 2 of the present Embodiment 2, the barrel 212 and the liquid collection needle 220 can be separated by irreversibly breaking at the boundary between the base end 221 and the small-diameter portion 216. That is, in FIGS. 5A and 5B, each of the barrel 212 and the liquid collection needle 220 are grasped with different hands, and when a bending force or a pulling force is applied thereto, the boundary portion between the base end 221 and the small-diameter portion 216 is broken. The rib 22 formed on the liquid collection needle 20 of Embodiment 1 is not formed in the present Embodiment 2. For this reason, when the bending force is applied, stress is concentrated at the boundary portion between the base end 221 and the small-diameter portion 216, and it is possible to easily perform breaking at the boundary portion. FIG. 7A is a perspective view of the injector 2 (injector main body 210) from which the liquid collection needle 220 has been thus separated and removed, and FIG. 7B is a cross-sectional view thereof. As shown in FIG. 7B, a break mark 216a that is formed due to the liquid collection needle 220 being removed is formed at the inner circumferential end of the small-diameter portion 216. The injector main body 210 from which the liquid collection needle 220 has been removed is substantially the same as the injector main body 10 (see FIGS. 1A and 1B) of Embodiment 1, except that it includes the break mark 216a.

It is possible to use the same materials as those of the injector 1 of Embodiment 1 as the materials of the portions constituting the injector 2. In the present Embodiment 2, the barrel 212 and the liquid collection needle 220 are manufactured integrally as one part overall through an extrusion molding method or the like.

### Method of Use (said method is not forming part of the invention)

The injector 2 can be used in the case of performing the above-described simple suspension method. A simple suspension method using the injector 2 is performed as follows.

Firstly, the injector 2 in which the liquid collection needle 220 is integral with the barrel 212 as shown in FIGS. 5A and 5B is prepared.

Next, the leading end of the liquid collection needle 220 is immersed in the medicinal liquid in which a tablet has been disintegrated, the plunger 218 is operated, and the medicinal liquid is suctioned into the barrel 212. The suction amount of the medicinal liquid is measured using the position of the gasket 19, which can be seen through the barrel 212, and the notches (not shown) on the barrel 212.

Next, the liquid collection needle 220 is separated and removed from the barrel 212 (see FIGS. 7A and 7B).

Thereafter, similarly to Embodiment 1, the tube end 14 is connected to the male connector 910 (patient-side connector, see FIGS. 21A and 21B) and the medicinal liquid in the liquid storing portion 15 is administered to the patient.

### Effect

According to the present Embodiment 2, the task (suctioning task) of suctioning the medicinal liquid in the container into the injector main body 210 is performed in a state in which the liquid collection needle 220 is connected to the barrel 212, and thereafter, the task (administration task) of administering the medicinal liquid in the injector main body 210 to the patient is performed with the liquid collection needle 220 separated and removed and the tube end 14 connected to the male connector 910. Accordingly, similarly to Embodiment 1, in the suction task, the medicinal liquid does not attach to the outer circumferential surface of the tubular portion 921 including the spiral projection 925. For this reason, when the tube end 14 is connected to the male connector 910 thereafter, the medicinal liquid does not attach to the inner circumferential surface (in particular, the female screw 915) of the outer tube 913 of the male connector 910. Accordingly, it is possible to prevent the male connector 910 from reaching an unhygienic state, even if the male connector 910 is left in the patient for a long time.

Before the liquid collection needle 220 is separated from the barrel 212, the base end 221 of the liquid collection needle 220 and the small-diameter portion 216 of the barrel 212 are fluid-tightly connected (see FIG. 6). For this reason, similarly to Embodiment 1, the medicinal liquid in the liquid storing portion 15 does not pass between the base end 221 and the small-diameter portion 216 to leak to the tube end 14 side. Accordingly, when the simple suspension method is performed using the injector 2, the medicinal liquid does not remain in the tube end 14. For this reason, in the present Embodiment 2, the likelihood that an amount of the medicinal liquid that is greater than the measured amount will be administered to the patient is reduced, regardless of the worker. Accordingly, the injector 2 of the present Embodiment 2 is advantageous for accurately managing a medicinal liquid amount to be administered to the patient.

With the injector 2 of the present Embodiment 2, the barrel 212 and the liquid collection needle 220 are one part, and therefore it is possible to reduce the number of members constituting the injector 2, which makes it easier to manufacture the injector 2.

Also, once the liquid collection needle 220 is separated from the barrel 212, the liquid collection needle 220 cannot be re-connected to the barrel 212. Accordingly, the injector 2 of the present Embodiment 2 is a so-called disposable type which cannot be re-used. Since the simple suspension method will always be performed using a new and clean injector 2, the likelihood that the patient will be infected is further reduced.

### Embodiment 3

### Configuration

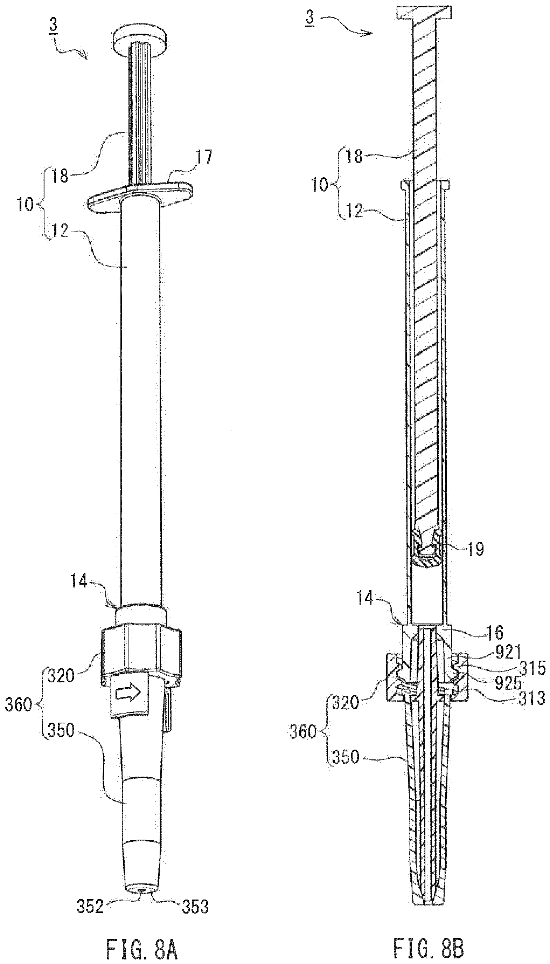

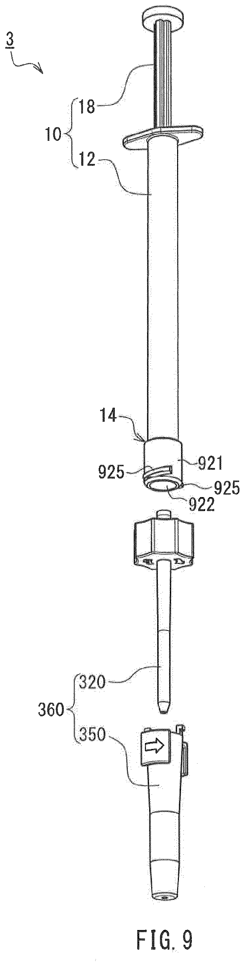

FIG. 8A is a perspective view of an injector 3 according to Embodiment 3 of the present invention. FIG. 8B is a cross-sectional view of the injector 3. FIG. 9 is an exploded perspective view of the injector 3. As shown in FIG. 9, the injector 3 includes the injector main body 10 having the barrel (outer tube) 12 and the plunger 18, a liquid collection needle 320, and a nozzle tip 350. The liquid collection needle 320 and the nozzle tip 350 constitute a liquid collection nozzle 360. The injector main body 10 of the present Embodiment 3 is the same as the injector main body 10 of Embodiment 1. Among the members constituting the injector 3 of Embodiment 3, members that are the same as the members constituting the injector 1 of Embodiment 1 are denoted by the same reference numerals as in Embodiment 1, and detailed description thereof is omitted.

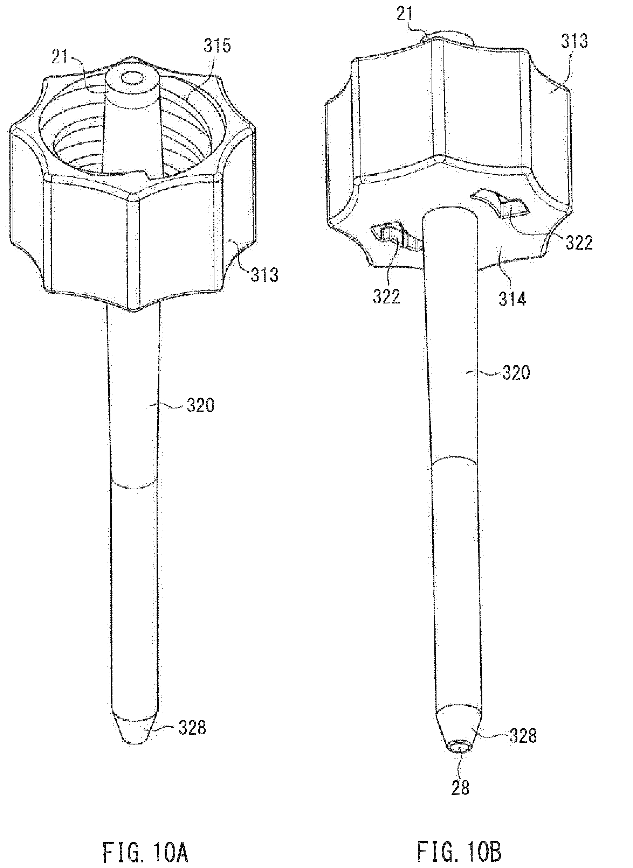

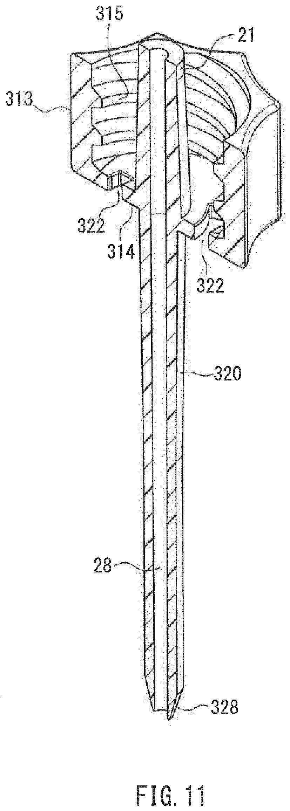

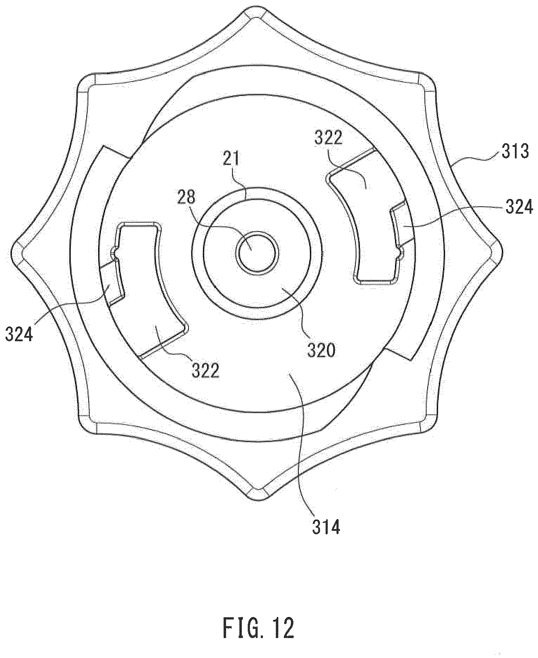

FIG. 10A is a perspective view from above of the liquid collection needle 320 and FIG. 10B is a perspective view from below of the liquid collection needle 320. FIG. 11 is a cross-sectional perspective view of the liquid collection needle 320 and FIG. 12 is a plan view of the liquid collection needle 320. The liquid collection needle 320 of the present Embodiment 3 is provided with an outer tube 313 that surrounds the liquid collection needle 320 near the collection tube 21. The liquid collection needle 320 and the outer tube 313 are joined via a bottom plate 314 that protrudes in a flange shape along the radial direction from the liquid collection needle 320. A female screw 315 is provided on the inner circumferential surface opposing the liquid collection needle 320 of the outer tube 313. The female screw 315 is interchangeable with the female screw 915 (see FIGS. 21A and 21B) provided on the above-described male connector 910 and conforms to ISO 80369-3. Accordingly, the female screw 315 can be screwed on the spiral projection (male screw) 925 provided on the tube end 14 of the barrel 12 conforming to ISO 80369-3. An approximately octagonal prism surface is provided on the outer circumferential surface of the outer tube 313 such that it is easy to grasp the outer tube 313 and apply a rotational force to the liquid collection tip 320. Note that the shape of the outer circumferential surface of the outer tube 313 is not limited thereto and can be changed as appropriate.

As shown in FIG. 12, two through holes 322 that are approximately L-shaped in plan view are provided on the bottom plate 314. The two through holes 322 have 180-degree rotational symmetry with respect to the central axis of the liquid collection needle 320. Inclined surfaces 324 are provided near the edges of the through holes 322 on the upper surface of the bottom plate 314. The inclined surfaces 324 are inclined so as to rise toward the clockwise direction with respect to the liquid collection needle 320.

As shown in FIG. 10A, a tapered surface (male tapered surface) 328 with an outer diameter that becomes smaller as the leading end is approached is provided on the outer circumferential surface near the leading end of the liquid collection tip 320.

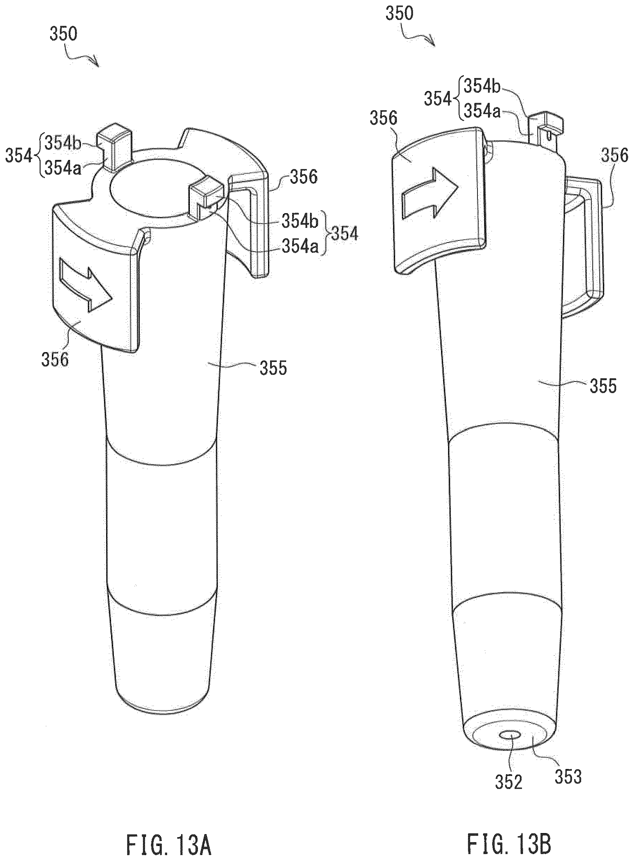

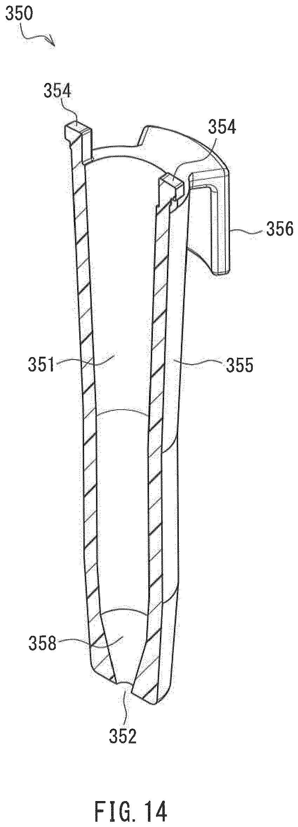

FIG. 13A is a perspective view from above of the nozzle tip 350, FIG. 13B is a perspective view from below of the nozzle tip 350, and FIG. 14 is a cross-sectional perspective view of the nozzle tip 350.

As shown in FIG. 14, the nozzle tip 350 has a hollow, approximately cylindrical shape. The inner diameter of the inner circumferential surface that defines an inner cavity 351 of the nozzle tip 350 is set to be the same as or greater than the outer diameter of the portion below the bottom plate 314 of the liquid collection needle 320 (see FIGS. 8A and 8B) so that the portion of the liquid collection needle 320 can be inserted. The inner diameter of the nozzle tip 350 is at its minimum at the opening 352 provided on the leading end thereof (the lower end). The inner diameter of the opening 352 is preferably set to be about the same as the inner diameter of the opening (see FIG. 10B) at the leading end of the flow path 28 of the liquid collection needle 320. A tapered surface (female tapered surface) 358 with an inner diameter that becomes smaller as the opening 352 is approached is provided adjacent to the opening 352 and on the upper side thereof. The female tapered surface 358 has the same tapering angle and diameter as the male tapered surface 328 (see FIG. 10A) provided near the leading end of the liquid collection needle 320.

As shown in FIG. 13A, two projections 354 are provided on the upper end of the nozzle tip 350. Each projection 354 includes a vertical portion 354a that extends upward from the upper end of the nozzle tip 350 and an engagement portion 354b that protrudes outward along the radial direction from the upper end of the vertical portion 354a. The two projections 354 have 180-degree rotational symmetry with respect to the central axis of the nozzle tip 350. Furthermore, two grasping portions 356 are provided on the nozzle tip 350. The grasping portions 356 each protrude outward along the radial direction from the upper end of the nozzle tip 350, and thereafter extend downward. The grasping portions 356 are provided in order to make it easier to apply a rotational force to the nozzle tip 350. The shapes of the grasping portions 356 are not limited to the present embodiment. For example, it is also possible to provide a regular polygonal prism surface (a regular hexagonal prism surface, a regular octagonal prism surface, or the like) on the outer circumferential surface of the nozzle tip 350 and use this as the grasping portion. The grasping portions may also be omitted.

As shown in FIG. 13B, a tapered surface (male tapered surface) 355 with an outer diameter that becomes smaller as the leading end is approached is provided on the outer circumferential surface of the nozzle tip 350. A circular plane 353 that is perpendicular to the lengthwise direction of the nozzle tip 350 is provided on the leading end of the nozzle tip 350. The outer diameter of the leading end surface 353 is larger than the outer diameter of the leading end (see FIG. 10B) of the liquid collection needle 320. The opening 352 is provided in the center of the leading end surface 353. The outer circumferential edge of the leading end surface 353 is smoothly chamfered.

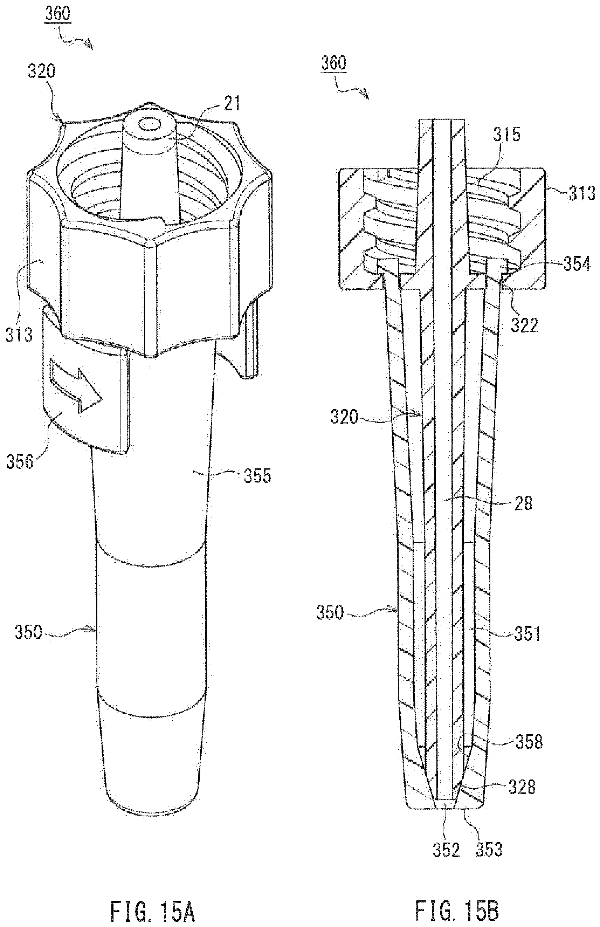

The nozzle tip 350 can be repeatedly detachably mounted on the liquid collection needle 320. FIG. 15A is a perspective view of the liquid collection nozzle 360 in which the nozzle tip 350 is mounted on the liquid collection needle 320. FIG. 15B is a cross-sectional view of the liquid collection nozzle 360.

The nozzle tip 350 is mounted on the liquid collection needle 320 overall as follows. The liquid collection needle 320 is inserted into the inner cavity 351 of the nozzle tip 350 and the projections 354 of the nozzle tip 350 are inserted into the through holes 322 provided on the bottom plate 314 of the liquid collection needle 320. In this state, the liquid collection needle 320 and the nozzle tip 350 are rotated in mutually opposite directions (i.e., in a view from above, the liquid collection needle 320 is rotated in the counterclockwise direction with respect to the nozzle tip 350). Engagement portions 354b of the nozzle tip 350 (see FIGS. 13A and 13B) slide on the inclined surfaces 324 (see FIG. 12) of the liquid collection needle 320. Since the inclined surfaces 324 are inclined as described above, the liquid collection needle 320 moves relatively along the lengthwise direction with respect to the nozzle tip 350 such that the liquid collection needle 320 is more deeply inserted into the cavity 351 of the nozzle tip 350 as the nozzle tip 350 rotates with respect to the liquid collection needle 320. The nozzle tip 350 is rotated with respect to the liquid collection needle 320 until the vertical portions 354a of the projections 354 of the nozzle tip 350 come into contact with the trailing ends in the rotational direction of the edges that define the through holes 322 of the liquid collection needle 320. FIGS. 15A and 15B show this state. The male tapered surface 328 of the liquid collection needle 320 and the female tapered surface 358 of the nozzle tip 350 are fit together fluid-tightly and air-tightly. The leading end of the liquid collection needle 320 is stored in the nozzle tip 350 and is covered by the nozzle tip 350. The opening 352 on the leading end of the nozzle tip 350 and the flow path 28 of the liquid collection needle 320 are in communication.

The liquid collection needle 320 and the nozzle tip 350 can be separated by performing an operation opposite to that described above.

The nozzle tip 350 can be easily rotated relative to the liquid collection needle 320 by grasping each of the outer circumferential surface of the outer tube 313 of the liquid collection needle 320 and the grasping portion 356 of the nozzle tip 350 with different hands and applying a rotational force.

The material of the liquid collection needle 320 is not limited, and it is possible to use the same material as that of the liquid collection needle 20 described in Embodiment 1. Since the nozzle tip 350 directly touches the skin of a person, a material with a relatively low hardness is preferable, and specifically, it is possible to use a resin material such as polypropylene (PP) or polyethylene (PE). The liquid collection needle 320 and the nozzle tip 350 can be manufactured integrally as one part overall through an extrusion molding method or the like, using the above-described resin materials.

### Method of Use (said method is not forming part of the invention)

The injector 3 of the present embodiment can be used for breast milk extraction, in addition to being able to be used in the simple suspension method similarly to the injectors 1 and 2 of Embodiments 1 and 2.

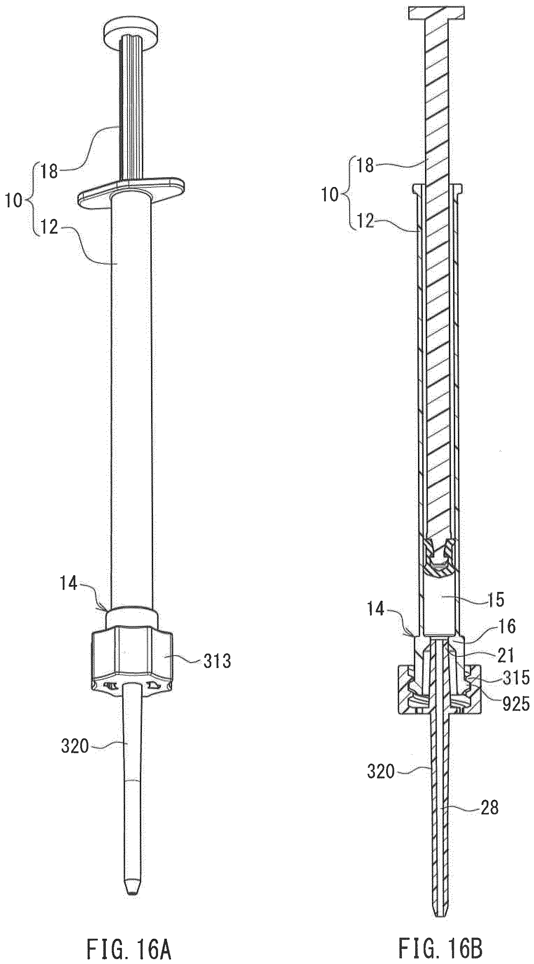

Firstly, the simple suspension method using the injector 3 will be described. In the case of performing the simple suspension method, the nozzle tip 350 is removed from the injector 3 shown in FIGS. 8A and 8B. That is, as shown in FIGS. 16A and 16B, only the liquid collection needle 320 is mounted on the injector main body 10. As shown in FIG. 16B, similarly to Embodiment 1, the connection tube 21 of the liquid collection needle 320 is fit into the small-diameter portion 16 of the barrel 12 and the connection tube 21 and the small-diameter portion 16 are fluid-tightly connected. The flow path 28 of the liquid collection needle 320 is in communication with the liquid storing portion 15 of the barrel 12. The female screw 315 of the liquid collection needle 320 is screwed onto the spiral projection 925 of the barrel 12.

Next, the leading end of the liquid collection needle 320 is immersed in the medicinal liquid in which a tablet has been disintegrated, the plunger 18 is operated, and the medicinal liquid is suctioned into the barrel 12. Next, the liquid collection needle 320 is taken off of the tube end 14 (see FIG. 9). Thereafter, similarly to Embodiment 1, the tube end 14 is connected to the male connector 910 (see FIGS. 21A and 21B) and the medicinal liquid in the liquid storing portion 15 is administered to the patient. Thereafter, the tube end 14 is separated from the male connector 910.

Next, breast milk extraction using the injector 3 will be described. As shown in FIGS. 8A and 8B, breast milk is extracted in a state in which the liquid collection needle 320 and the nozzle tip 350 are mounted on the injector main body 10. The leading end surface 353 of the nozzle tip 350 is placed against the nipple, the plunger 18 is operated, and breast milk attached to the nipple is suctioned into the barrel 12 via the opening 352.

### Effect

According to the present Embodiment 3, in the case of performing a simple suspension method, the task (suction task) of suctioning the medicinal liquid in the container into the injector main body 10 is performed with the liquid collection needle 320 mounted on the tube end 14, and on the other hand, the task (administration task) of administering the medicinal liquid in the injector main body 10 to the patient is performed with the tube end 14 connected to the male connector 910, without using the liquid collection needle 320. Accordingly, similarly to Embodiment 1, in the suction task, the medicinal liquid does not attach to the outer circumferential surface of the tubular portion 921 including the spiral projection 925. For this reason, when the tube end 14 is connected to the male connector 910 thereafter, the medicinal liquid does not attach to the inner circumferential surface (in particular, the female screw 915) of the outer tube 913 of the male connector 910. Accordingly, it is possible to prevent the male connector 910 from reaching an unhygienic state, even if the male connector 910 is left in the patient for a long time.

When the liquid collection needle 320 is mounted on the tube end 14 (see FIGS. 16A and 16B), the connection tube 21 of the liquid collection needle 20 and the small-diameter portion 16 of the barrel 12 are fluid-tightly connected. For this reason, similarly to Embodiment 1, the medicinal liquid in the liquid storing portion 15 does not pass between the connection tube 21 and the small-diameter portion 16 to leak to the tube end 14 side. Accordingly, the injector 3 of the present Embodiment 3 is advantageous for accurately managing a medicinal liquid amount to be administered to the patient.

Furthermore, the injector 3 of the present Embodiment 3, on which the nozzle tip 350 is mounted, can be preferably used for breast milk extraction. In general, with breast milk extraction, breast milk is suctioned into the barrel in a state in which the tube end of the injector (syringe) is placed directly against the nipple. For example, breast milk extraction can be performed using the conventional injector 950 (see FIGS. 19A and 19B) on which the liquid collection tip 940 is not mounted. A circular plane with a relatively large area is provided on the leading end of the tube end 954, and a flow path is opened in the center thereof. The circular plane is placed against the nipple and the breast milk is suctioned. The nozzle tip 350 of the present Embodiment 3 includes a leading end surface 353 that is equal to or larger than the leading end surface of the tube end 954, and therefore the leading end surface 353 can be placed against the nipple and breast milk extraction can be performed.

In contrast to this, the leading ends of the liquid collection needles 20, 220, and 320 of Embodiments 1 to 3 have relatively small diameters so as to be able to perform extraction without leaving even a small amount of fluid in the container. If the leading ends of the liquid collection needles 20, 220, and 320 are placed directly against the nipple, the mother may feel pain. On the other hand, since a cavity with a large inner diameter exists in the tubular portion 921 of the tube end 14 of the injector main bodies 10 and 210 (see FIGS. 2 and 7A) from which the liquid collection needles 2, 220, and 320 have been removed, it is difficult to suction a small amount of breast milk. The nozzle tip 350 of the injector 3 of the present Embodiment 3 has a leading end surface 353 with a relatively large diameter and large area, and therefore no pain is felt when the leading end of the nozzle tip 350 directly touches the skin. Small amounts of breast milk can be suctioned with little remaining liquid by bringing the leading end surface 353 of the nozzle tip 350 into close contact with the nipple.

Thus, according to the present Embodiment 3, it is possible to perform both the simple suspension method and breast milk extraction using the same injector main body 10.

With the present Embodiment 3, the nozzle tip 350 is used while mounted on the liquid collection needle 320. Unlike the present Embodiment 3, a configuration is conceivable in which a breast milk extraction nozzle having an outer shape similar to that of the nozzle tip 350 is created, and the breast milk extraction nozzle is mounted on the tube end 14 instead of the liquid collection needle 320 in the case of performing breast milk extraction. However, in this configuration, the thickness in the radial direction of the breast milk extraction nozzle needs to be increased. This kind of thick breast milk extraction nozzle generally has low resin formability. In contrast to this, the nozzle tip 350 of the present embodiment can be made thinner, and therefore has excellent resin formability.

When the nozzle tip 350 is mounted on the liquid collection needle 320, the female tapered surface 358 of the nozzle tip 350 and the male tapered surface 328 of the liquid collection needle 320 fit together near the opening 352 of the nozzle tip 350, and an air-tight and fluid-tight seal is formed between the two surfaces (see FIG. 15B) When the plunger 18 is pulled while the leading end surface 353 is pressed against the nipple, the seal prevents the external air from flowing into the flow path 28 of the liquid collection needle 320 through the gap between the nozzle tip 350 and the liquid collection needle 320 from the opening on the upper end (the outer tube 313 side) of the nozzle tip 350. For this reason, the breast milk can be suctioned into the barrel 12 through the flow path 28 of the liquid collection needle 320 from the opening 352 of the nozzle tip 350. Also, among the breast milk that flows into the opening 352 of the nozzle tip 350, the amount of breast milk that flows to the gap between the nozzle tip 350 and the liquid collection needle 320 and does not flow in the flow path 28 can be reduced. Accordingly, the seal between the liquid collection needle 320 and the nozzle tip 350 is advantageous for efficiently extracting the breast milk. The inclined surfaces 324 of the liquid collection needle 320 (see FIG. 12) are advantageous for improving the sealing property between the liquid collection needle 320 and the nozzle tip 350.

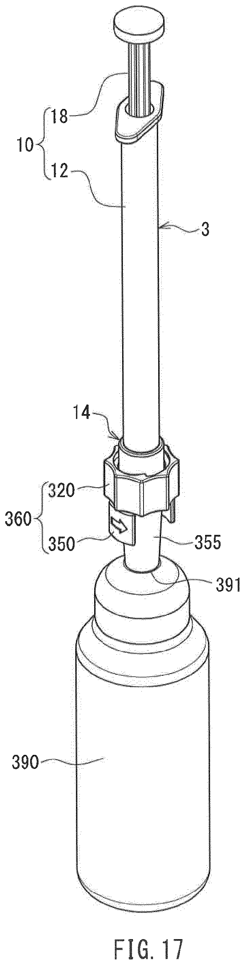

The male tapered surface 355 is provided on the outer circumferential surface of the nozzle tip 350 (see FIG. 13A). For this reason, as shown in FIG. 17 for example, the injector 3 can pierce the open opening 391 of the container (e.g., a distilled water container) 390. The male tapered surface 355 of the nozzle tip 350 is fit into the edge of the opening 391 of the container 390, and the opening 391 is closed by the nozzle tip 350. Temporarily leaving the injector 3 and the container 390 in this state is advantageous for preventing contamination of the leading end of the nozzle tip 350 and preventing contamination and evaporation of the liquid (e.g., the distilled water) in the container 390. However, in the present invention, the male tapered surface 355 is not essential.

The liquid collection needle 320 includes the outer tube 313 (see FIG. 10A). In the state in which the liquid collection needle 320 is mounted on the barrel 12 (see FIGS. 8A, 8B, 16A, and 16B), the outer tube 313 covers the tube end 14 of the barrel 12 (in particular, the spiral projection 925 and the periphery thereof). The outer tube 313 prevents the tube end 14 from being contaminated by being touched by a finger of the worker or the like. Accordingly, the outer tube 313 is advantageous for ensuring the hygienic state of the tube end 14. Accordingly, the hygienic state of the male connector 910 to which the tube end 14 is connected can be kept favorable, and contamination of the patient can be prevented. Also, the worker can attach/detach the liquid collection needle 320 to/from the tube end 14 of the barrel 12 without touching the leading end of the liquid collection needle 320 by holding the outer circumferential surface of the outer tube 313. The task of mounting the liquid collection nozzle 360 (see FIG. 15A) in which the nozzle tip 350 has been mounted on the liquid collection needle 320 on the tube end 14 of the barrel 12 can also be performed by similarly holding the outer circumferential surface of the outer tube 313.

The female screw 315 that can be screwed onto the spiral projection 925 of the barrel 12 is provided on the outer tube 313. For this reason, although the liquid collection needle 320 does not include the fitting shape 22 (see FIG. 3A) that fits into the inner circumferential surface 922 of the tubular portion 921 of the barrel 12, which is provided in the liquid collection needle 20 of Embodiment 1, it is possible to firmly mount the liquid collection needle 320 on the barrel 12.

The female screw 315 provided in the liquid collection needle 320 and the spiral projection 925 provided in the barrel 12 are so-called right screws. In contrast to this, the structure for engaging the projections 354 of the nozzle tip 350 and the bottom plate 314 of the liquid collection needle 320 is configured such that when the liquid collection needle 320 is rotated in the counterclockwise direction with respect to the nozzle tip 350 in a view from above, engagement occurs, and when the liquid collection needle 320 is rotated in the clockwise direction with respect to the nozzle tip 350, the engagement is canceled, and the relationship between the engagement, the canceling thereof, and the rotation directions is the same as that of a so-called left screw. Accordingly, in the state in which the liquid collection needle 320 and the nozzle tip 350 are mounted on the barrel 12 as shown in FIGS. 8A and 8B, when each of the barrel 12 and the nozzle tip 350 are grasped with a different hand and the barrel 12 is rotated in the counterclockwise direction with respect to the nozzle tip 350 in a view from the barrel 12 side, the nozzle tip 350 can be separated from the liquid collection needle 320 without loosening the screwing of the spiral projection 925 of the barrel 12 and the female screw 315 of the liquid collection needle 320. Thus, by providing the engagement structure conforming to a left screw between the liquid collection needle 320 and the nozzle tip 350, even if one of the barrel 12 and the outer tube 313 of the liquid collection needle 320 is grasped with one hand in the state shown in FIGS. 8A and 8B, the nozzle tip 350 can be reliably removed from the liquid collection needle 320 if the nozzle tip 350 is grasped with the other hand and rotated as described above.

The above-described Embodiment 3 is merely an example. The configuration of the above-described Embodiment 3 of the present invention can be modified as needed.

For example, as a lock mechanism for stably maintaining the state in which the nozzle tip 350 is mounted on the liquid collection needle 320, the above-described Embodiment 3 included an engagement structure in which the engagement portions 354b of the nozzle tip 350 are engaged with the bottom plate 314 of the liquid collection needle 320. However, the lock mechanism is not limited to this kind of engagement structure, and any configuration can be employed.

For example, the lock mechanism may be a screw structure. In an example, it is possible to provide a spiral projection (e.g., a male screw) on the outer circumferential surface of the liquid collection needle 320 and to provide a female screw that screws on the spiral projection on the inner circumferential surface near the upper end of the nozzle tip 350. In another example, the outer tube 313 of the liquid collection needle 320 may extend below the bottom surface 314, a female screw may be provided on the inner circumferential surface of the extended outer tube 313, and a screw projection (e.g., a male screw) that screws into the female screw may be provided on the outer circumferential surface near the upper end of the nozzle tip 350. The screw structure can be advantageous for improving the sealing property between the liquid collection needle 320 and the nozzle tip 350. The fact that the screw structure constituting the lock mechanism is configured to conform to a left screw is advantageous for reliably removing the nozzle tip 350 from the liquid collection needle 320 without loosening the screwing between the spiral projection 925 and the female screw 315 in a state in which the liquid collection needle 320 and the nozzle tip 350 are mounted on the barrel 12 as shown in FIGS. 8A and 8B, similarly to the above-described engagement structure of Embodiment 3.

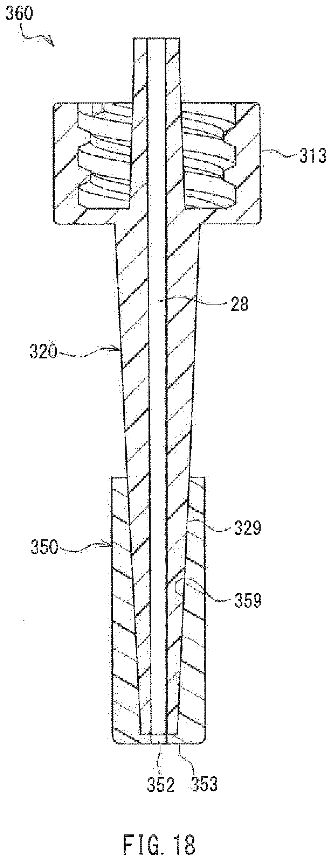

Alternatively, the above-described lock mechanism may be omitted. For example, as shown in FIG. 18, a tapered surface (male tapered surface) 329 with an outer diameter that becomes smaller as the leading end is approached is provided on the outer circumferential surface of the liquid collection needle 320, and a female tapered surface 359 with the same diameter and tapering angle as the male tapered surface 329 is provided on the inner circumferential surface of the nozzle tip 350. It is possible to stably hold the nozzle tip 350 on the liquid collection needle 320 with the frictional force between the male tapered surface 329 and the female tapered surface 359 when they are fit together. An air-tight and fluid-tight seal is formed between the male tapered surface 329 and the female tapered surface 359. In this case, the nozzle tip 350 may be constituted by a soft material that deforms relatively easily, such as a material having rubber elasticity (also referred to as an elastomer), for example, a rubber such as natural rubber, isoprene rubber, or silicone rubber, or a thermoplastic elastomer such as styrene-based elastomer, olefin-based elastomer, or polyurethane-based elastomer.

In the above-described Embodiment 3, an air-tight and fluid-tight seal was formed between the male tapered surfaces 328 and 329 of the liquid collection needle 320 and the female tapered surfaces 358 and 359 of the nozzle tip 350, but the seal between the liquid collection needle 320 and the nozzle tip 350 may be formed by surfaces other than the two fit-together tapered surfaces. For example, the air-tight and fluid-tight seal between the leading end surface (surface surrounding the opening on the leading end side of the flow path 28) of the liquid collection needle 320 and the inner surface (surface on the side opposite to that of the plane 353) of the leading end of the nozzle tip 350 may be formed by bringing them into contact in the lengthwise direction of the liquid collection needle 320.

The above-described liquid collection needle 320 included the female screw 315 that can screw onto the spiral projection 925 of the barrel 12 on the inner circumferential surface of the outer tube 313, but it is possible to omit the female screw 315. Furthermore, it is also possible to omit the female screw 315 and the outer tube 313. The liquid collection needle 320 may include a fitting shape that fits in the inner circumferential surface of the tubular portion 921 of the barrel 12, which was described in Embodiment 1.

The leading end surface 353 of the nozzle tip 350 does not need to be a precise flat surface. For example, it may protrude or be recessed in a dome shape. However, it is preferable that the corners are chamfered in a round shape so that no sharp edges are included.

The nozzle tip that covers the leading end of the liquid collection needle described in the present Embodiment 3 may be applied to the liquid collection needles 20 and 220 of Embodiments 1 and 2.

In Embodiments 1 and 3, the shape of the outer circumferential surface of the connection tube 21 is not limited to being a cylindrical surface. The shape of the connection tube 21 is arbitrary, as long as it is possible to fluid-tightly connect to the small-diameter portion 16. For example, the outer circumferential surface of the connection tube 21 may be a male tapered surface (conical surface) with an outer diameter that becomes smaller as the leading end is approached. Instead of fitting the connection tube 21 into the small-diameter portion 16, the connection tube 21 may be fluid-tightly connected to the small-diameter portion 16 by coming into contact with the small-diameter portion 16, for example. Alternatively, the connection tube 21 may be fluid-tightly connected to the small-diameter portion 16 by forming a tube-shaped projection that protrudes downward (into the tubular portion 921) on the small-diameter portion 16 and fitting the tube-shaped projection into the connection tube 21.

Although a case was described in which a simple suspension method is performed using the liquid collection needles 20, 220, and 320 in the above-described Embodiments 1 to 3, the liquid collection needle of the present invention can be used also to suction any liquid (water, medicinal liquid, blood, etc.) into the injector using a method other than the simple suspension method.

### Industrial Applicability

The present invention, although not limited, can be used widely in the field of medicine as an injector for collecting any liquid (water, medicinal liquid, breast milk, blood, etc.). In particular, the present invention can be used preferably as an injector to be used in the case of performing a simple suspension method.

### List of Reference Numerals

- 1,2,3: Medical liquid collection injector
- 12,212: Barrel
- 14: Tube end
- 15: Liquid storing portion
- 16,216: Small-diameter portion
- 18: Plunger
- 20,220,320: Liquid collection needle
- 21: Base end of liquid collection needle (connection tube)
- 22: Rib (fitting shape)
- 28: Flow path of liquid collection needle
- 221: Base end of liquid collection needle
- 350: Nozzle tip
- 352: Opening of nozzle tip
- 354: Projection (lock mechanism)
- 355: Male tapered surface of nozzle tip
- 360: Liquid collection nozzle
- 921: Tubular portion
- 922: Inner circumferential surface of tubular portion (female tapered surface)
- 925: Spiral projection (male screw)

## Claims

1. A medical liquid collection injector (1; 2; 3) comprising:
a tube-shaped barrel (12; 212) that includes an opening at one end and a tube end (14) at another end;
a plunger (18) inserted into the opening of the barrel; and
a tube-shaped liquid collection needle (20; 220; 320),
**characterised in that** the tube end includes a cylindrically-shaped tubular portion (921) and a spiral projection (925) that is formed on an outer circumferential surface of the tubular portion,
an inner circumferential surface (922) of the tubular portion is a female tapered surface with an inner diameter that becomes larger as a leading end is approached,
the barrel includes a small-diameter portion (16; 216) with an inner diameter that is relatively small between a liquid storing portion (15) in which the plunger is inserted and the tubular portion (921), and
a base end (21; 221) of the liquid collection needle (20; 220; 320) is fluid-tightly connected to the small-diameter portion (16; 216), and a leading end of the liquid collection needle protrudes from the tube end.

2. The medical liquid collection injector according to claim 1, wherein
the barrel (12) and the liquid collection needle (20; 320) are separate parts, and
the liquid collection needle is detachably mounted on the tube end (14).

3. The medical liquid collection injector according to claim 2, wherein a fitting shape (22) that fits in the inner circumferential surface (922) of the tubular portion (921) is formed on the liquid collection needle (20).

4. The medical liquid collection injector according to claim 1, wherein
the barrel (212) and the liquid collection needle (220) constitute a part that is formed integrally, and
the liquid collection needle and the barrel can be irreversibly separated at a boundary (216a) between the base end (221) and the small-diameter portion (216).

5. The medical liquid collection injector according to any one of claims 1 to 4, further comprising a nozzle tip (350) that is to be detachably mounted on the medical liquid collection needle (320) so as to cover at least the leading end of the liquid collection needle,
wherein the nozzle tip includes an opening (352) that is provided so as to communicate with a flow path (28) of the liquid collection needle when the nozzle tip is mounted on the liquid collection needle.

6. The medical liquid collection injector according to claim 5, wherein an air-tight and fluid-tight seal is formed between the liquid collection needle (320) and the nozzle tip (350) when the nozzle tip is mounted on the liquid collection needle.

7. The medical liquid collection injector according to claim 5 or 6, wherein a lock mechanism (322, 354) for maintaining a state in which the nozzle tip (350) is mounted on the liquid collection needle (320) is provided.

8. The medical liquid collection injector according to any one of claims 5 to 7, wherein a male tapered surface (355) with an outer diameter that becomes smaller as a leading end is approached is provided on the outer circumferential surface of the nozzle tip (350).

## Patentansprüche

1. Medizinischer Flüssigkeitssammelinjektor (1; 2; 3), Folgendes umfassend:
einen rohrförmigen Körper (12; 212), der eine Öffnung an einem Ende und ein Rohrende (14) an dem anderen Ende einschließt;
einen Kolben (18), der in die Öffnung des Körpers eingeführt ist; und
eine rohrförmige Flüssigkeitssammelnadel (20; 220; 320),
**dadurch gekennzeichnet, dass** das Rohrende einen zylindrisch geformten rohrförmigen Abschnitt (921) und eine Spiralprojektion (925) einschließt, die an einer Außenumfangsoberfläche des rohrförmigen Abschnitts gebildet ist,
eine Innenumfangsoberfläche (922) des rohrförmigen Abschnitts eine spitz zulaufende Buchsenoberfläche mit einem Innendurchmesser ist, der mit Annäherung an ein vorderes Ende größer wird,
der Körper einen Abschnitt mit kleinem Durchmesser (16; 216) mit einem Durchmesser einschließt, der zwischen einem Flüssigkeitsaufbewahrungsabschnitt (15), in den der Kolben eingeführt ist, und dem rohrförmigen Abschnitt (921) relativ klein ist, und
ein Basisende (21; 221) der Flüssigkeitssammelnadel (20; 220; 320) fluiddicht mit dem Abschnitt mit kleinem Durchmesser (16; 216) verbunden ist und ein vorderes Ende der Flüssigkeitssammelnadel aus dem Rohrende vorspringt.

2. Medizinischer Flüssigkeitssammelinjektor nach Anspruch 1, wobei
der Körper (12) und die Flüssigkeitssammelnadel (20; 320) getrennte Teile sind, und die Flüssigkeitssammelnadel abnehmbar an dem Rohrende (14) montiert ist.

3. Medizinischer Flüssigkeitssammelinjektor nach Anspruch 2, wobei eine angepasste Form (22), die in die Innenumfangsoberfläche (922) des rohrförmigen Abschnitts (921) passt, auf der Flüssigkeitssammelnadel (20) gebildet wird.

4. Medizinischer Flüssigkeitssammelinjektor nach Anspruch 1, wobei
der Körper (212) und die Flüssigkeitssammelnadel (220) ein Teil bilden, das in einem Stück gebildet ist, und
die Flüssigkeitssammelnadel und der Körper unumkehrbar an einer Grenze (216a) zwischen dem Basisende (221) und dem Abschnitt mit kleinem Durchmesser (216) getrennt werden können.

5. Medizinischer Flüssigkeitssammelinjektor nach einem der Ansprüche 1 bis 4, weiter umfassend eine Düsenspitze (350), die abnehmbar an der medizinischen Flüssigkeitssammelnadel (320) zu montieren ist, um mindestens das vordere Ende der Flüssigkeitssammelnadel abzudecken,
wobei die Düsenspitze eine Öffnung (352) einschließt, die bereitgestellt ist, um mit einem Strömungspfad (28) der Flüssigkeitssammelnadel zu kommunizieren, wenn die Düsenspitze auf der Flüssigkeitssammelnadel montiert ist.

6. Medizinischer Flüssigkeitssammelinjektor nach Anspruch 5, wobei eine luftdichte und fluiddichte Dichtung zwischen der Flüssigkeitssammelnadel (320) und der Düsenspitze (350) gebildet wird, wenn die Düsenspitze auf der Flüssigkeitssammelnadel montiert ist.

7. Medizinischer Flüssigkeitssammelinjektor nach Anspruch 5 oder 6, wobei ein Verriegelungsmechanismus (322, 354) zum Beibehalten eines Zustands, in dem die Düsenspitze (350) auf der Flüssigkeitssammelnadel (320) montiert ist, bereitgestellt wird.

8. Medizinischer Flüssigkeitssammelinjektor nach einem der Ansprüche 5 bis 7, wobei eine spitz zulaufende Steckoberfläche (355) mit einem Außendurchmesser, der mit Annäherung an ein vorderes Ende kleiner wird, an der Außenumfangsoberfläche der Düsenspitze (350) bereitgestellt wird.

## Revendications

1. Collecteur/injecteur de liquide à usage médical (1 ; 2 ; 3) comprenant :
un cylindre en forme de tube (12 ; 212) qui inclut une ouverture au niveau d'une extrémité et une extrémité de tube (14) au niveau d'une autre extrémité ;
un piston (18) inséré dans l'ouverture du cylindre ; et
une aiguille de collecte de liquide en forme de tube (20 ; 220 ; 320),
**caractérisé en ce que** l'extrémité de tube inclut une partie tubulaire de forme cylindrique (921) et une saillie en spirale (925) qui est formée sur une surface circonférentielle extérieure de la partie tubulaire,
une surface circonférentielle intérieure (922) de la partie tubulaire est une surface conique femelle avec un diamètre intérieur qui devient plus grand tandis qu'une extrémité avant est approchée,
le cylindre inclut une partie de petit diamètre (16 ; 216) avec un diamètre intérieur qui est relativement petit entre une partie de stockage de liquide (15) dans laquelle le piston est inséré et la partie tubulaire (921), et
une extrémité de base (21; 221) de l'aiguille de collecte de liquide (20; 220; 320) est raccordée de manière étanche aux fluides à la partie de petit diamètre (16 ; 216), et une extrémité avant de l'aiguille de collecte de liquide fait saillie depuis l'extrémité de tube.

2. Collecteur/injecteur de liquide à usage médical selon la revendication 1, dans lequel
le cylindre (12) et l'aiguille de collecte de liquide (20; 320) sont des parties séparées, et
l'aiguille de collecte de liquide est montée de manière détachable sur l'extrémité de tube (14).

3. Collecteur/injecteur de liquide à usage médical selon la revendication 2, dans lequel une forme d'ajustement (22) qui s'ajuste dans la surface circonférentielle intérieure (922) de la partie tubulaire (921) est formée sur l'aiguille de collecte de liquide (20).

4. Collecteur/injecteur de liquide à usage médical selon la revendication 1, dans lequel
le cylindre (212) et l'aiguille de collecte de liquide (220) constituent une partie qui est formée d'un seul tenant, et
l'aiguille de collecte de liquide et le cylindre peuvent être séparés de manière irréversible au niveau d'une limite (216a) entre l'extrémité de base (221) et la partie de petit diamètre (216).

5. Collecteur/injecteur de liquide à usage médical selon l'une quelconque des revendications 1 à 4, comprenant en outre une pointe de buse (350) qui doit être montée de manière détachable sur l'aiguille de collecte de liquide à usage médical (320) de manière à recouvrir au moins l'extrémité avant de l'aiguille de collecte de liquide,
dans lequel la pointe de buse inclut une ouverture (352) qui est pourvue de manière à communiquer avec un trajet d'écoulement (28) de l'aiguille de collecte de liquide lorsque la pointe de buse est montée sur l'aiguille de collecte de liquide.

6. Collecteur/injecteur de liquide à usage médical selon la revendication 5, dans lequel un joint étanche à l'air et étanche aux fluides est formé entre l'aiguille de collecte de liquide (320) et la pointe de buse (350) lorsque la pointe de buse est montée sur l'aiguille de collecte de liquide.

7. Collecteur/injecteur de liquide à usage médical selon la revendication 5 ou 6, dans lequel un mécanisme de verrouillage (322, 354) pour maintenir un état dans lequel la pointe de buse (350) est montée sur l'aiguille de collecte de liquide (320) est pourvu.

8. Collecteur/injecteur de liquide à usage médical selon l'une quelconque des revendications 5 à 7, dans lequel une surface conique mâle (355) avec un diamètre extérieur qui devient plus petit tandis qu'une extrémité avant est approchée est pourvue sur la surface circonférentielle extérieure de la pointe de buse (350).
